# FASCICULE DE BREVET EUROPEEN

(11) **EP 1 638 547 B1**
(45) Date de publication et mention de la délivrance du brevet: **24.01.2007**
(21) Numéro de dépôt: 04767382.7
(22) Date de dépôt: 18.06.2004
(51) Int. Cl.: A61K 31/201, A61K 36/00, A61K 35/60, A61P 13/00, A61K 45/06

(54) **COMPOSITION POUR LA PREVENTION DES INFECTIONS DU SYSTEME URINAIRE**
ZUSAMMENSETZUNG ZUR PRÄVENTION VON HARNWEGSINFEKTIONEN
COMPOSITION FOR PREVENTING URINARY SYSTEM INFECTIONS

(30) Priorité: 20.06.2003 FR 0307465
(43) Date de publication de la demande: 29.03.2006
(73) Titulaire: Natural Product Consulting, 6700 Strasbourg (FR)
(72) Inventeur: ANTON, Jean-Christophe, F-67000 Strasbourg (FR)
(74) Mandataire: Honoré, Anne-Claire
(86) Numéro de dépôt international: PCT/FR2004/001523
(87) Numéro de publication internationale: WO 2004/112775

(56) Documents cités:
- WO-A-01/76568
- WO-A-02/02105
- WO-A-97/29763
- WO-A-99/12541
- US-A1- 2001 012 525
- US-A1- 2002 068 100
- US-A1- 2003 026 859
- ROY ET AL: "Traditional herbal therapy using a mixture of Allium sativa (GARLIC) and Nigella sativa (KALAJIRA) in the treatment of urinary tract infections" BANGLADESH RENAL JOURNAL, vol. 11, no. 1, 1992, pages 13-17, XP008039304

## Description

La présente invention concerne des compositions pour la prévention des infections du système urinaire, compositions qui sont par conséquent essentiellement utilisées à titre prophylactique.

Les infections urinaires comprennent d'une part la colonisation microbienne asymptomatique de l'urine, et d'autre part l'infection symptomatique avec invasion microbienne et inflammation des structures de l'arbre urinaire. Les bactéries sont de loin les micro-organismes infectant les plus fréquents, mais des champignons et virus peuvent également produire une infection urinaire. Les micro-organismes proliférant et les cellules inflammatoires présentes dans l'urine constituent la caractéristique biologique de cette affection.

Les infections urinaires sont une des pathologies les plus fréquentes rencontrées par les médecins, en grande majorité chez les femmes, en particulier sous forme de cystite, ou inflammation de la vessie. Celles-ci entraînent des récidives infectieuses qui peuvent être fréquentes. Dans ce cas, les traitements antibiotiques disponibles sont efficaces, mais souvent mal tolérés, et ils engendrent de plus des résistances croissantes de la part des germes uropathogènes. A propos de la montée en puissance des résistances aux antibiotiques, des voies s'élèvent d'ailleurs dans le monde médical pour encourager les médecins à s'interroger plus systématiquement sur la pertinence de l'utilisation d'antibiotiques.

Dans nombre de cas, les sujets atteints d'infections urinaires récurrentes souffrent d'une baisse importante de leur qualité de vie et d'une extrême fatigue pouvant conduire à la dépression. L'apparition, ces dernières années, de résistances aux antibiotiques conduit à augmenter la fréquence des rechutes, et diminue encore la qualité de vie des patients et tout en augmentant le coût économique de ces pathologies pour la société. Pour toutes ces raisons, espacer les récurrences, en d'autres termes éviter les récidives, devient un objectif prioritaire à la fois pour les sujets souffrant de ces pathologies et pour la communauté scientifique.

Les principaux moyens de défense naturelles contre ces infections urinaires relèvent du volume et de la dynamique du flux urinaire (environ 1,5 1 par jour), permettant des vidanges régulières et complètes de la vessie (4 à 5 fois par jour), ainsi que des propriétés antibactériennes de l'épithélium bordant l'appareil urinaire (urothélium). L'intégrité et l'imperméabilité de l'urothélium qui recouvre les cavités urinaires est donc particulièrement important. Parmi d'autres moyens, on peut citer la sécrétion d'une protéine particulière (Tamm-Horsfall) secrétée par le rein et présente dans les urines, ainsi que les sécrétions vaginales chez la femme et prostatiques chez l'homme.

En général, le traitement actuellement préconisé d'une infection urinaire récidivante ne diffère pas de celui d'un épisode unique. Pour les sujets présentant plus de trois infections par an, certains traitements prophylactiques basés sur des médicaments de type antibiotique sont prévus. Mais à part des mesures d'hygiène et un traitement sur plusieurs années par des doses modérées d'antibiotiques, il n'y a pas aujourd'hui de traitement préventif satisfaisant à long terme des infections urinaires, et en particulier des cystites.

Compte tenu des limitations mentionnées qui semblent émerger à propos des antibiothérapies, le traitement préventif des infections urinaires doit impérativement prendre des voies nouvelles.

La demande de brevet WO 97/29763 propose ainsi des compositions visant à traiter les troubles urogénitaux et intestinaux, lesquelles compositions contiennent une quantité efficace d'au moins une plante de la famille Ericaceae, de préférence Vaccinium macrocarpon, ou un extrait de ladite plante, et une quantité efficace d'un facteur de croissance stimulant spécifiquement la croissance des bactéries acido-lactiques.

Les compositions peuvent optionnellement contenir de l'ail. Les compositions selon ce document agissent contre les germes pathogènes à l'origine des infections urinaires combattues.

La présente invention propose une autre solution de mise en commun: d'actifs permettant de prévenir la récurrence des infections urinaires. La combinaison: de ces actifs va permettre une action préventive plus efficace que la même action d'un seul d'entre eux pris séparément, car ils interagissent.

Plus précisément, la composition pour la prévention des infections du système urinaire selon l'invention est caractérisée en ce qu'elle contient des composants prévus pour exercer des actions simultanées en vue de :
- supprimer les germes pathogènes à l'origine de ces infections ou réduire leur nocivité ;
- renforcer l'intégrité de la paroi urinaire.

En d'autres termes, le rôle de ces actifs est d'agir simultanément en vue du même objectif (empêcher les réinfections) mais par des voies d'approche différentes : une action directe sur les germes, et une action indirecte sur la paroi du système urinaire.

En ce qui concerne la première action, la composition de l'invention comporte, en vue de la suppression et/ou de la réduction de la nocivité des germes pathogènes, des composants visant à exercer une action antibactérienne directement sur les germes présents dans les voies urinaires, mais également sur les germes présents dans le tractus gastro-intestinal. Par cette dernière action, les composants visent à réduire les possibilités de colonisation des voies urinaires par des germes provenant du tractus digestif et ainsi limiter les infections. La composition de l'invention comporte également des composants pour diminuer l'aptitude des germes à adhérer aux parois du système urinaire.

Dans l'étape d'adhésion aux voies urinaires par le germe pathogène infectant, dans la majorité des cas Escherichia coli, les facteurs bactériens qui influencent l'adhérence initiale entre le germe et la muqueuse urogénitale impliquent l'expression de pili (comme P-fimbriae, localisés sur la membrane d'Escherichia coli) qui sont sur-représentés dans des isolats de germes de patients atteints de cystites. L'adhérence implique également l'interaction entre ce pili et des uroplakin I situés sur la paroi de la vessie. C'est ce mécanisme qui est le promoteur de l'adhésion et qu'il s'agit de faire diminuer..

Dans l'optique de renforcer l'intégrité de la paroi du système urinaire, des composants de l'invention permettent à la fois de diminuer l'inflammation de la paroi, et de rééquilibrer son système immunitaire.

L'intégrité de épithélium tapissant ladite paroi est un des éléments clé permettant à l'organisme de lutter contre l'infection. En effet, Escherichia coli est incapable de se fixer sur la muqueuse intacte des parois du système urinaire. En revanche, des dommages au niveau de cette muqueuse permettent aux germes un accès aux récepteurs présents sur l'urothélium sous-jacent, et conduisent ainsi à l'adhérence des germes sur ces parois.

Pour se défendre, l'épithélium est capable de générer une exfoliation lui permettant de se débarrasser des germes adhérant aux cellules. Pour ce faire, il faut préalablement diminuer l'inflammation de la paroi, une paroi infectée ne pouvant mettre en oeuvre dans de bonnes conditions ces moyens de défense en présence de dommages au niveau de l'épithélium.

Des dommages sont également produits du fait de perturbations du système immunitaire, qui favorisent par conséquent également la colonisation de l'épithélium par des germes pathogènes.

Dans ce cas, les mastocytes, cellules immunitaires largement impliquées dans les cystites en particulier, produisent des altérations de l'intégrité de la muqueuse par le processus de l'inflammation. C'est la raison pour laquelle la régulation du système immunitaire et la diminution de l'inflammation vont de paire dans le renforcement de l'intégrité de la paroi.

Selon l'invention, les agents antibactériens aboutissant à supprimer les germes pathogènes ou à réduire une nocivité sont choisis dans le groupe des composés soufrés du type alliicine .

L' alliicine a en effet démontré des propriétés antibactériennes, antifongiques et antivirales sur des souches d'agents pathogènes parfois résistantes aux thérapies- conventionnelles, c'est-à-dire basées sur des antibiotiques. Expérimentalement, la sensibilité de différentes préparations de bactéries et de virus à cette substance est d'ailleurs très significative.

De plus, il n'y a pas de phénomène de résistance à l'alliicine car son mode d'action est complètement différent de celui des antibiotiques.

Dans l'article de DL. LAMM et D.R. RIGGS, Enhanced Immunocompetence by Garlic : Role in Bladder Cancer and other Malignancies, Journal of Nutrition, vol.131, no. 13s, 2001, pages 1067s-1070s, il est ainsi démontré que l'activité antitumorale de l'aïl est au moins en partie liée à une stimulation immunitaire : phagocytose accrue, augmentation des cellules tueuses (NK) et immunoprolifération des lymphocytes sont les principaux phénomènes observés.

Les agents antibactériens soufrés dérivés de l'alliicine sont extraits de plantes du genre Allium, et plus précisément des variétés Allium sativum et Allium cepa.

L'ail, employé depuis l'Antiquité comme antiseptique, a une activité antibactérienne et antifongique qui a été mise en évidence in-vitro. Ces propriétés ont été reconnues de longue date par Pasteur, et l'ail a été utilisé empiriquement dans le monde entier pour traiter les infections microbiennes.

Les plantes de la famille de l'ail, et plus particulièrement la variété Allium cepa, montrent un spectre antibiotique large sur des bactéries gram + et gram négatif. Le jus d'ail est quant à lui efficace sur la plupart des bactéries pathogènes du tube digestif, même sur les souches résistantes aux antibiotiques.

Selon l'invention, le ou les composants visant à réduire l'adhésion des germes à la paroi sont des anthocyanosides, des acides organiques et des dérivés polyphénoliques extraits d'un au moins parmi les genres. Ericaceae, Saxifragaceae, Vitaceae et Caprifoliaceae et/ou des fructoses de fruits.

Selon une possibilité, lesdits composants peuvent être extraits de la famille Ericaceae, et plus précisément du genre Vaccinium et des espèces Vaccinium myrtillus, Vaccinium corymbosum et Vaccinium macrocarpon.

En prenant l'exemple du Vaccinium macrocarpon, cette espèce septentrionale croit spontanément dans l'Est de l'Amérique du Nord, des Carolines au Canada. Il produit des petits fruits rouge foncé, largement consommés en nature ainsi que sous forme de jus. L'effet bénéfique, bactériostatique du jus de fruit dans le traitement des infections urinaires est confirmé par un usage très ancien. Il semble que son action soit liée à une inhibition de l'adhérence des bactéries sur les muqueuses. Cela a été démontré dans le cas de l'adhésion de l'Escherichia coli sur des cellules uroépithéliales.

Le brevet WO 99/12541 propose à cet égard certains extraits purifiés de proanthocyanidines de plantes de la famille Ericaceae, notamment et du genre Vaccinium en particulier, qui sont capables d'inhiber sélectivement l'adhérence des bactéries E.Coli de type P, impliquées dans les infections rénales, et pas celle des bactéries E. Coli de type 1, impliquées dans les infections de la vessie.

Les compositions comportant de tels extraits sont destinées au traitement des infections du conduit urinaire.

Selon certaines études, cette plante aurait des vertus de prévention de récurrence des infections urinaires, et serait donc une alternative intéressante aux autres thérapies visant à espacer les récurrences.

Alternativement, selon l'invention, ces composants peuvent être extraits de la famille Saxifragaceae, et plus précisément de l'espèce Ribes nigrum.

En variante encore, ils peuvent être extraits de la famille Vitaceae, et plus précisément de l'espèce Vitis vinifera.

Alternativement encore, ils peuvent être extraits du genre. Caprifoliaceae, et plus précisément du Sambucus nigra.

Par ailleurs, selon une autre de ses caractéristiques principales, la composition de l'invention contient des acides gras polyinsaturés de la famille n-3, visant à améliorer les mécanismes de défense de la muqueuse.

Le précurseur de la famille des acides gras polyinsaturés n-3 est l'acide linoléique, que l'on retrouve dans les tissus de plantes vertes et dans certaines huiles d'origine végétale. Dans l'organisme, l'acide linoléique est converti en acides eicosapentaénoique (EPA) et docosahexaénoique (DHA).

L'alimentation des pays développés est environ 10 fois plus riche en acide linolénique, précurseur de la famille des acides gras polyinsaturés n-6, qu'en acide linoléique, et le métabolisme du premier domine par conséquent dans le corps humain.

Ainsi, nos cellules ne contiennent que de faibles quantités de EPA et de DHA suite aux déséquilibres alimentaires précités. Or, il a été constaté de manière surprenante que certaines populations qui consomment des quantités importantes de poisson et d'huile de poisson, qui sont riches en EPA et en DHA, on une très faible incidence de maladies inflammatoires et auto-immmunes. Ceci suggère en particulier que ces acides gras s'insèrent à la place de l'acide arachidonique, précurseur des prostaglandines et des leukotriènes qui ont de fortes propriétés pro-inflammatoires. Cette insertion permet ainsi d'inhiber de manière compétitive la conversion de l'acide arachidonique en prostaglandines. Ceci s'accompagne de la diminution de la production par les cellules inflammatoires des médiateurs de l'inflammation comme les prostaglandines et les leukotriènes. EPA et DHA vont également agir sur certains mécanismes du système immunitaire et rétablir ainsi son équilibre, évitant ainsi la dégranulation importante des mastocytes et les dommages créés au niveau de l'urothélium.

Ainsi, dans l'invention, l'acide gras poly-insaturé est choisi parmi l'acide eicosapentaénoique (EPA) et l'acide docosahexaénoique (DHA).

Plus précisément encore, l'acide eicosapentaénoique étant présent dans l'huile de poisson, la composition pour la prévention des infections du système urinaire selon l'invention contient une telle huile de poisson.

Selon une possibilité résultant des choix possibles tels que mentionnés auparavant, la composition de l'invention est caractérisée en ce qu'elle contient :
- du Vaccinium macrocarpon ;
   de l'Allium sativum ; et
   de l'huile de poisson.

Plus précisément, elle est conditionnée de manière à contenir :
de 100 à 1000 mg d'extrait de Vaccinium macrocarpon ;
de 10 à 1000 mg d'extrait d'Allium sativum ; et
de 500 à 2000 mg d'huile de poisson.

Bien entendu, le choix du conditionnement ou des composants de la composition de l'invention, ainsi que les poids relatifs desdits composants, dépendent de la sélection des composants effectuée sur la base des indications données dans la présente description. Ainsi, chacun du Vaccinium macrocarpon, de l'allium sativum et de l'huile de poisson peut être remplacé par des composants équivalents, mentionnés auparavant selon un dosage qui n'est néanmoins pas celui qui a été donné sur ces trois composants et qui fait partie du savoir-faire de l'homme de l'art.

## Revendications

1. Composition pour la prévention des infections du système urinaire, **caractérisée en ce que** :
- en vue de la suppression des germes pathogènes et/ou la réduction de leur nocivité, elle comprend :
- des composés soufrés dérivés d'alliicine extraits de plantes genre Allium, et plus précisément les variétés Allium sativum et Allium cepa, visant à exercer une action antibactérienne sur les germes présents dans les voies urinaires et le tractus gastro-intestinal, de façon à diminuer la colonisation par ces derniers des voies urinaires, et
- des composants choisis dans le groupe constitué des anthocyanosides et des dérivés polyphénoliques, extraits d'au moins une parmi les familles Ericaceae, Saxifragaceae, Vitaceae et Caprifoliaceae et/ou des fructoses de fruits, en tant que composants qui visent à réduire l'adhésion des germes à la paroi,
et **en ce que**
- en vue du renforcement de l'intégrité de la paroi des voies urinaires, elle comprend des acides gras polyinsaturés de la famille n-3 permettant de diminuer l'inflammation de celle-ci et de rééquilibrer le système immunitaire.

2. Composition pour la prévention des infections du système urinaire selon la revendication 1, **caractérisée en ce que** lesdits composants pour réduire l'adhésion des germes à la paroi sont extraits de la famille Ericaceae et plus précisément du- genre Vaccinium et des espèces Vaccinium myrtillus, Vaccinium corymbosum et Vaccinium macrocarpon.

3. Composition pour la prévention des infections du système urinaire selon la revendication 1, **caractérisée en ce que** lesdits composants pour réduire l'adhésion des germes à la paroi sont extraits de la famille Saxifragaceae et plus précisément de l'espèce Ribes nigrum.

4. Composition pour la prévention des infections du système urinaire selon la revendication 1, **caractérisée en ce que** lesdits composants pour réduire l'adhésion des germes à la paroi sont extraits de la famille Vitaceae et plus précisément de l'espèce Vitis vinifera.

5. Composition pour la prévention des infections du système urinaire selon la revendication 1, **caractérisée en ce que** lesdits composants pour réduire l'adhésion des germes à la paroi sont extraits du genre Caprifoliaceae et plus précisément du Sambucus nigra.

6. Composition pour la prévention des infections du système urinaire selon la revendication 1, **caractérisée en ce que** l'acide gras polyinsaturé est choisi parmi l'acide eicosapentaénoique (EPA) et l'acide docosahexaénoique (DHA).

7. Composition pour la prévention des infections du système urinaire selon la revendication 1, **caractérisée en ce qu'**elle contient de l'huile de poisson en tant que substance comprenant au moins un acide gras polyinsaturé.

8. Composition pour la prévention des infections du système urinaire selon l'une quelconque des revendications précédentes, **caractérisée en ce qu'**elle contient :
du Vaccinium macrocarpon ;
de l'Allium sativum ; et
de l'huile de poisson.

9. Composition pour la prévention des infections du système urinaire selon la revendication précédente, **caractérisée en ce qu'**elle est conditionnée de manière à contenir :
de 100 à 1000 mg d'extrait de Vaccinium macrocarpon ;
de 10 à 1000 mg d'extrait d'Allium sativum ; et
de 500 à 2000 mg d'huile de poisson.

## Claims

1. Composition for preventing urinary system infections, **characterized in that**:
- in order to suppress pathogenic germs and/or to reduce their noxiousness, it comprises:
- sulfurated compounds derived from allicin, extracted from plants of the genus *Allium,* and more specifically the varieties *Allium sativum* and *Allium cepa*, with the aim of exerting anti-bacterial action onto germs present in the urinary tracts and gastro-intestinal tract, so as to reduce colonization by the latter germs of the urinary tracts, and
- components selected from the group consisting of anthocyanosides and polyphenol derivatives, extracted from at least one of the families *Ericaceae, Saxifragaceae, Vitaceae* and *Caprifoliaceae* and/or fructoses from fruit, as components which aim at reducing adhesion of the germs to the wall,
and **in that**
- in order to reinforce the integrity of the wall of the urinary tracts, it comprises polyunsaturated fatty acids from the n-3 family allowing the inflammation of the latter to be reduced and the immune system to be brought back into balance.

2. The composition for preventing urinary system infections according to claim 1, **characterized in that** said components for reducing the adhesion of the germs to the wall are extracted from the *Ericaceae* family, and more specifically from the *Vaccinium* genus, and from the *Vaccinium myrtillus, Vaccinium corymbosum* and *Vaccinium macrocarpon* species.

3. The composition for preventing urinary system infections according to claim 1, **characterized in that** said components for reducing the adhesion of the germs to the wall are extracted from the *Saxifragaceae* family and more specifically from the *Ribes nigrum* species.

4. The composition for preventing urinary system infections according to claim 1, **characterized in that** said components for reducing the adhesion of the germs to the wall are extracted from the *Vitaceae* family and more specifically from the *Vitis vinifera* species.

5. The composition for preventing urinary system infections according to claim 1, **characterized in that** said components for reducing the adhesion of the germs to the wall are extracted from the *Caprifoliaceae* genus and more specifically from *Sambucus nigra*.

6. The composition for preventing urinary system infections according to claim 1, **characterized in that** the polyunsaturated fatty acid is selected from eicosapentaenoic acid (EPA) and docosahexaenoic acid (DHA).

7. The composition for preventing urinary system infections according to claim 1, **characterized in that** it contains fish oil as a substance comprising at least one polyunsaturated fatty acid.

8. The composition for preventing urinary system infections according to any of the preceding claims, **characterized in that** it contains:
*Vaccinium macrocarpon;*
*Allium sativum;* and
fish oil.

9. The composition for preventing urinary system infections according to the preceding claim, **characterized in that** it is conditioned in order to contain:
from 100 to 1,000 mg of *Vaccinium macrocarpon* extract;
from 10 to 1,000 mg of *Allium sativum* extract; and
from 500 to 2,000 mg of fish oil.

## Patentansprüche

1. Zusammensetzung zur Prävention von Harnwegsinfektionen, **dadurch gekennzeichnet, dass**:
- sie im Hinblick auf die Unterdrückung pathogener Keime und/oder die Verringerung ihrer Schädlichkeit umfasst:
- Schwefelverbindungen, abgeleitet von Alliicin, gewonnen aus Pflanzen der Gattung *Allium*, und genauer der Varietäten *Allium sativum* und *Allium cepa,* die darauf abzielen, eine antibakterielle Wirkung auf die in den Harnwegen und im Magen-Darm-Trakt vorhandenen Keime auszuüben, um die Besiedlung der Harnwege durch sie zu verringern, und
- Bestandteile, ausgewählt aus der Gruppe, die von Anthocyanosiden und Polyphenolderivaten gebildet wird, die mindestens aus einer der Familien Ericaceae, Saxifragaceae, Vitaceae und Caprifoliaceae und/oder aus Fruktosen von Früchten gewonnen werden, als Bestandteile, die darauf abzielen, die Adhäsion der Keime an der Wand zu verringern,
und **dadurch**, dass
- sie im Hinblick auf die Stärkung der Integrität der Wand der Harnwege mehrfach ungesättigte Fettsäuren der Familie n-3 umfasst, die es erlauben, ihre Entzündung zu verringern und das Immunsystem wieder ins Gleichgewicht zu bringen.

2. Zusammensetzung zur Prävention von Harnwegsinfektionen nach Anspruch 1, **dadurch gekennzeichnet, dass** diese Bestandteile zur Verringerung der Adhäsion der Keime an der Wand aus der Familie Ericaceae und genauer aus der Gattung *Vaccinium* und der Arten *Vaccinium myrtillus, Vaccinium corymbosum* und *Vaccinium macrocarpon* gewonnen werden.

3. Zusammensetzung zur Prävention von Harnwegsinfektionen nach Anspruch 1, **dadurch gekennzeichnet, dass** diese Bestandteile zur Verringerung der Adhäsion der Keime an der Wand aus der Familie Saxifragaceae und genauer aus der Art *Ribes nigrum* gewonnen werden.

4. Zusammensetzung zur Prävention von Harnwegsinfektionen nach Anspruch 1, **dadurch gekennzeichnet, dass** diese Bestandteile zur Verringerung der Adhäsion der Keime an der Wand aus der Familie Vitaceae und genauer aus der Art *Vitis vinifera* gewonnen werden.

5. Zusammensetzung zur Prävention von Harnwegsinfektionen nach Anspruch 1, **dadurch gekennzeichnet, dass** diese Bestandteile zur Verringerung der Adhäsion der Keime an der Wand aus der Gattung Caprifoliaceae und genauer aus *Sambucus nigra* gewonnen werden.

6. Zusammensetzung zur Prävention von Harnwegsinfektionen nach Anspruch 1, **dadurch gekennzeichnet, dass** die mehrfach ungesättigte Fettsäure aus der Eicosapentaensäure (EPA) und der Docosahexaensäure (DHA) ausgewählt ist.

7. Zusammensetzung zur Prävention von Harnwegsinfektionen nach Anspruch 1, **dadurch gekennzeichnet, dass** sie Fischöl als Substanz enthält, die mindestens eine mehrfach ungesättigte Fettsäure aufweist.

8. Zusammensetzung zur Prävention von Harnwegsinfektionen nach einem der vorhergehenden Ansprüche, **dadurch gekennzeichnet, dass** sie enthält:
*Vaccinium macrocarpon,*
*Allium sativum*, und
Fischöl.

9. Zusammensetzung zur Prävention von Harnwegsinfektionen nach dem vorhergehenden Anspruch, **dadurch gekennzeichnet, dass** sie derart konditioniert ist, dass sie enthält:
100 bis 1000 mg Extrakt aus *Vaccinium macrocarpon,*
10 bis 1000 mg Extrakt aus *Allium sativum,* und
500 bis 2000 mg Fischöl.
